# EUROPÄISCHE PATENTSCHRIFT

(11) **EP 2 001 440 B1**
(45) Veröffentlichungstag und Bekanntmachung des Hinweises auf die Patenterteilung: **10.02.2010**
(21) Anmeldenummer: 07711959.2
(22) Anmeldetag: 15.03.2007
(51) Int. Cl.: A61K 9/127, A61K 31/155, A61P 31/02

(54) **Polihexanidhaltige Liposomen**
Polihexanide containing liposomes
Liposomes contenant du polihexanide

(30) Priorität: 01.04.2006 DE 102006015271
(43) Veröffentlichungstag der Anmeldung: 17.12.2008
(73) Patentinhaber: Lohmann & Rauscher GmbH & Co. KG, 56567 Neuwied (DE)
(72) Erfinder: ROHRER, Christian, A-4020 Linz (AT); WILHELMS, Tim, Axel, 56068 Koblenz (DE); WAGNER, Andreas, A-2500 Baden (AT)
(74) Vertreter: Flaccus, Rolf-Dieter
(86) Internationale Anmeldenummer: PCT/EP2007/002287
(87) Internationale Veröffentlichungsnummer: WO 2007/115635

(56) Entgegenhaltungen:
- EP-A1- 0 509 338
- EP-A1- 0 613 685
- WO-A-02/36257
- WO-A-92/20319
- FARKAS E ET AL: "Effect of beta-sitosterol on the characteristics of vesicular gels containing chlorhexidine" INTERNATIONAL JOURNAL OF PHARMACEUTICS (KIDLINGTON), Bd. 278, Nr. 1, 18. Juni 2004 (2004-06-18), Seiten 63-70, XP005204873 ISSN: 0378-5173

## Beschreibung

Die vorliegende Erfindung betrifft biguanidhaltige Liposomen, antiseptische Zubereitungen auf Basis von Liposomen, die mindestens ein Biguanid als mikrobioziden Wirkstoff enthalten, die Herstellung der biguanidhaltigen Liposomen und dieser antiseptischen Zubereitungen sowie ihre Verwendungsmöglichkeiten und die aus ihrer Verwendung hervorgehenden Produkte.

Der Heilungsprozeß einer Wunde verschlechtert sich bei einem Austrocknen der Wunde, einer hohen Bakterienzahl in der Wunde und/oder ihrer Behandlung mit toxischen Agentien deutlich. Dagegen fördern ein feuchtes Milieu, fehlende Keimbesiedlung und das vermehrte Vorhandensein von Wachstumsfaktoren die Epithelisierung und Bildung von Granulationsgeweben. Die Vorteile der feuchten Wundheilung, bei der ein feuchtes Milieu in der Wunde angestrebt wird, um das Zellwachstum zu stimulieren und so die schnellstmögliche Regeneration des geschädigten Gewebes zu erreichen, sind in Fachkreisen unbestritten. Allerdings schafft das feuchte Milieu zugleich gute Vermehrungsbedingungen für Bakterien. Eine mikrobielle Kontamination von Wunden stört deren Wundheilungsverlauf jedoch erheblich und stellt gerade in der jüngeren Zeit durch die zunehmende Resistenz von Mikroorganismen gegenüber Antibiotika ein immer größer werdendes Problem dar.

Da eine geringgradige Kolonisation von Wunden mit Mikroorganismen (umgangssprachlich "Mikroben") den Regelfall darstellt, sollte eine Ausbreitung der mikrobiellen Kontamination von Wunden verhindert oder nach Möglichkeit vermieden werden. Insbesondere eine Kontamination mit multiresistenten Erregern, z. B. Methicillin-resistenter *Staphylococcus aureus* (MRSA), ist zu behandeln, um ihre weitere Ausbreitung zu verhindern.

Mit Mikroorganismen verunreinigte bzw. infizierte Wunden sollten daher antiseptisch behandelt werden, weil
- sich eine Infektion entwickeln kann, solange die Wunde von Mikroorganismen besiedelt ist;
- der Wundheilungsprozeß nicht oder nur verzögert zum Abschluß kommt, solange die Wunde infiziert ist;
- sich die Wundinfektion ausbreiten und zu einer Sepsis führen kann; und
- bei einer Wundkolonisation mit multiresistenten Erregern eine Weiterverbreitung dieser Erreger verhindert werden muß.

Die Notwendigkeit einer frühzeitigen Prävention von Woundinfektionen, insbesondere bei zu erwartender Kontamination großer Wandflächen, besteht insbesondere auch bei Verbrennungswuaden.

In der Fachliteratur sind verschiedene Verfahren beschrieben worden, um Wunden auch von Mikroorganismen zu säubern, die gegen Antibiotika resistent sind. Beispielsweise wurde vorgeschlagen, Beläge und grobe Verschmutzungen mit Hilfe von Spüllösungen von der Wunde zu entfernen. Hierfür werden üblicherweise Ringer-Lösungen, Ringer-Lactat-Lösungen oder isotonische Kochsalz-Lösungen verwendet. Diese Lösungen besitzen jedoch keine antiseptische Wirkung, sie führen lediglich zu einer Reinigung der Wunde.

Lösungen zum Reinigen von Wunden, die eine antimikrobielle Wirkung haben, sind ebenfalls im Handel erhältlich. Es handelt sich bei diesen Produkten beispielsweise um Jod-Lösungen, Wasserstoffperoxid-Lösungen, Silbersalz-Lösungen oder Polyhexanid-Lösungen, die jedoch alle bestimmte Nachteile aufweisen.

Jod hat eine aggressive Oxidationswirkung, die zu einer zuverlässigen mikrobioziden Wirkung gegen grampositive und gramnegative Bakterien, Pilze und Protozoen führt, sowie gegen eine Reihe von Viren. Die Bereitstellung des wasserlöslichen Povidon-Jod (PVP-Jod, Polyvinylpyrrolidon-Jod) erlaubt zwar eine meist schmerzfreie Behandlung der Wunden gegenüber der Anwendung der davor gebräuchlichen alkoholhaltigen Jod-Lösungen (Jod-Tinktur), es gibt aber nach wie vor viele Kontraindikationen und Probleme bei der Anwendung von PVP-Jod. Beispielsweise wird Jod aus dem PVP-Jod über die Haut vom Organismus aufgenommen, es sind Jod-Allergien und Jod-Unverträglichkeiten bekannt, die auch der Anwendung von PVP-Jod entgegenstehen, und es wurde nachgewiesen, daß Jod die Zellteilung hemmt. Somit kommt es bei der Behandlung von Wunden mit PVP-Jod zu einer Verzögerung der Wundheilung.

Durch Einarbeitung von PVP-Jod in Liposomen, im Handel erhältlich unter der Bezeichnung Repithel® (Mundipharma GmbH, Limburg, DE), konnte die Gewebeverträglichkeit des PVP-Jod deutlich verbessert werden, ohne daß die Wirksamkeit des PVP-Jods beeinträchtigt ist. Die Grundsubstanz von Repithel® ist ein Polyacrylatgel, das neben Wasser sogenannte Hydrosomen, also spezielle mehrschichtige Liposomen, enthält. Diese zwiebelschalenförmig aus mehreren Phospholipid-Doppelschichten aufgebauten Liposomen enthalten neben PVP-Jod als niedrig dosiertes Antiseptikum auch eine große Menge Wasser. Dadurch kann Repithel® wie herkömmliche Hydrogel-Formulierungen Wasser abgeben und aufnehmen; es schafft auf diese Weise eine Feuchtigkeitsbalance.

Repithel® führt jedoch ebenso wie PVP-Jod an sich oder die altbekannte Jod-Tinktur zu einer meist vorübergehenden Färbung der behandelten Fläche. Zwar zeigt die braune Eigenfarbe des PVP-Jods die Wirksamkeit der PVP-Jod-haltigen Zubereitung an, führt aber auch zu einer Verunreinigung von Textilien. Bei der Anwendung von Repithel® wie auch allen anderen Jod-haltigen Zubereitungen sind zudem folgende Kontraindikationen zu beachten: hyperthyreote Schilddrüsenerkrankungen, Dermatitis herpetiformis Duhring, Überempfindlichkeit gegen Jod. Ferner ist von der Anwendung vor und nach einer Radiojodtherapie abzusehen. Auch während einer Schwangerschaft und der Stillzeit sowie bei Neugeborenen und Säuglingen bis zu einem Alter von 6 Monaten sollten Jod-haltige Zubereitungen nur nach äußerst sorgfältiger Abwägung und unter Kontrolle der Schilddrüsenfunktion durch einen Arzt angewendet werden.

Wasserstoffperoxid zerfällt in der Wunde rasch unter Freisetzung von Sauerstoff zu Wasser. Der freigesetzte Sauerstoff kann die Zellwände der kontaminierenden Bakterien oxidieren. Aufgrund seiner schäumenden Wirkung durch die rasche Sauerstoff-Freisetzung lassen sich insbesondere verschmutzte und/oder verkrustete Wunden mit Wasserstoffperoxid mechanisch gut reinigen. Allerdings führt die Behandlung mit Wasserstoffperoxid auch zu einer oberflächlichen Verätzung der Wunde, wodurch ihre Heilung zumindest hinausgezögert wird. Für eine Daueranwendung, insbesondere bei chronischen Wunden, ist Wasserstoffperoxid daher nicht geeignet.

Silbersalz-Lösungen wirken bakterizid, indem sie die bakterielle Zellwand zerstören und die bakteriellen Enzyme denaturieren. Problematisch ist jedoch die unzureichende Stabilität von Silbernitrat-Lösungen, die Möglichkeit einer Resorption von Silberionen und die Zerstörung der Hautoberfläche aufgrund der vom Silber verursachten Eiweißfällung. Daher gilt die Verwendung von Silbersalz-Lösungen zur Behandlung von Wunden in Fachkreisen seit längerem als überholt. Die Verwendung von Silbersulfadiazin, ein Komplex aus Silber und dem Sulfonamid Sulfadiazin, wird allein schon auf Grund des Antibiotikum-Anteils als nicht mehr vertretbar angesehen.

Applikationsfertige Polyhexanid-Lösungen zur Wundbehandlung sind im Handel unter den Namen Lavasept® (Fresenius AG, Bad Homburg, DE) oder Prontosan® W (B. Braun Petzold GmbH, Melsungen, DE) erhältlich. Polyhexanid (Polyhexamethylenbiguanid; PHMB) gilt als Lokalantiseptikum, das ein breites Wirkspektrum und eine gute Verträglichkeit aufweist. Polyhexanid wirkt über seine kationischen Ladungen antimikrobiell, indem es die Permeabilität der bakteriellen Zellmembran erhöht und über den damit verbundenen Verlust an Kalium und anderen Zytoplasmabestandteilen zum Zelltod führt. Aufgrund seiner vergleichsweise langsam eintretenden Wirkung wird Polyhexanid eher für wiederholte Anwendungen auf chronisch schlecht heilenden oder empfindlichen Wunden empfohlen.

Nachteilig bei der Verwendung von Polyhexanid-Lösungen ist jedoch, daß dieses Antiseptikum seine Wirksamkeit in Gegenwart bereits geringer Mengen negativ geladener Ionen, z. B. bei Anwesenheit von Alginat-, Acrylat-, Lactat- oder Jodidionen, verliert. Daher ist darauf zu achten, daß Polyhexanid-Lösungen nicht mit anderen Wundtherapeutika und/oder moderneren Wundverbänden gemeinsam angewendet wird. Auch bei der Auswahl von Wundabdeckungen ist auf deren wirkstofffreiheit zu achten.

Neben den antiseptischen Lösungen sind auch eine Reihe von Wundauflage verfügbar, die zusätzlich Wirkstoffe enthalten, welche die Auflage vor einer mikrobiellen Kolonisation schützen und die Keimzahl in der Wunde verringern sollen. Insbesondere Wundauflagen, die als antimikrobielle Ausrüstung Silber oder Silbersalze enthalten, sind verbreitet, beispielsweise die Produkte Actisorb® (Johnson & Johnson WM, Norderstedt, DE) und Contreet®-H (Coloplast GmbH, Hamburg, DE). Die Wundauflage Actisorb®, bei der es sich um eine Kombination von elementarem Silber und Aktivkohle handelt, wird speziell bei infizierten und exulzerierenden Wunden zur Beseitigung unangenehmer Gerüche verwendet. Bei Contreet®-H handelt es sich um einen Hydrokolloidverband mit eingeschlossenen Silberionen, der abhängig vom Exsudationsverhalten der Wunde antiseptische Silberkonzentrationen in der Wunde erzeugt.

Seit kurzer Zeit existieren auch Wundauflage auf Basis von Kollagen, Cellulosederivaten oder Alginaten, die als antimikrobiellen Wirkstoff Polyhexanid in Konzentrationen von meist 0,5 bis 2 % enthalten. Diese Wundauflagen werden durch Besprühen oder Imprägnieren des Basis- bzw. Trägermaterials mit einer wäßrigen, Polyhexanid enthaltenden Lösung hergestellt. Allerdings hat sich herausgestellt, daß Polyhexanid ausgezeichnet an die Basis- bzw. Trägermaterialien bindet, die üblicherweise für die Herstellung von Wundauflage und Verbänden verwendet werden. Dadurch ist die Freisetzung von Polyhexanid aus den Wundauflagen und damit einhergehend die antimikrobielle Wirkung des Polyhexanids beeinträchtigt.

Die der vorliegenden Erfindung zugrundeliegende Aufgabe bestand darin, eine antiseptische Zubereitung bereitzustellen, mit der Wunden gereinigt und/oder behandelt werden können, die ein breites Wirkungsspektrum und eine gute Verträglichkeit aufweist, die weder zu Denaturierungserscheinungen noch zu Verfärbungen der Wunde oder von Gegenständen führt, die bei der Behandlung der Wunde mit der Zubereitung oder mit der behandelten Wunde in Kontakt kommen, und die auch für Daueranwendungen bei z. B. chronischen Wunden geeignet ist.

Ein Ziel der Erfindung war, eine antiseptische Zusammensetzung bereitzustellen, mit der das Konzept der feuchten Wundbehandlung aufrecht erhalten werden kann, ohne daß eine Kontamination der Wunde oder einer zu verwendenden Wundauflage befürchtet werden muß.

Ein weiteres Ziel der Erfindung war, ein Verfahren zur Herstellung von Liposomen anzugeben, die stabil sind und mindestens ein Biguanid mit antimikrobieller Wirkung enthalten.

Ein weiteres Ziel der Erfindung war, antiseptische Wundauflagen bereitzustellen, die mindestens ein Biguanid mit antimikrobieller Wirkung umfassen, bei denen die Verfügbarkeit des Biguanids und damit einhergehend dessen antimikrobielle Wirkung verbessert ist.

Erfindungsgemäß werden diese Ziele durch die Bereitstellung von Liposomen bestimmter Zusammensetzung erreicht, die mindestens ein Biguanid mit antimikrobieller Wirkung enthalten.

So lassen sich stabile Liposomen in Gegenwart von Biguaniden herstellen, wenn bei der Herstellung der Liposomen auf Lipide verzichtet wird, die eine anionische Kopfgruppe aufweisen, beispielsweise Phosphatidylglycerol. Zur Herstellung und Beladung von Liposomen mit Biguaniden hat sich das in der WO 02/36257 beschriebene Crossflow-Injektionsverfahren aufgrund seiner sehr schonenden Verfahrensbedingungen und hohen Effizienz als besonders vorteilhaft herausgestellt. Die Herstellung von mit Biguaniden beladenen Liposomen ist jedoch nicht auf dieses Verfahren beschränkt. Auch andere im Stand der Technik bekannte Verfahren zur Herstellung und Beladung von Liposomen können dazu genutzt werden, beispielsweise Hochdruckhomogenisations-, Microfluidizer- oder Ultraschallverfahren.

Biguanide, die in den erfindungsgemäßen Liposomen inkorporiert sein können, sind vorzugsweise aus der Gruppe von pharmakologisch akzeptablen Biguaniden ausgewählt, die 1,1'-Hexamethylen-bis-(5-(4-chlorophenyl)-biguanid) (Chlorhexidin), 1,1'-Hexamethylen-bis-(5-(4-fluorophenyl)-biguanid) (Fluorhexidine), Polyhexamethylenbiguanid (PHMB), Alexidin (N,N"-Bis(2-ethylhexyl)-3,12-diimino-2,4,11,13-tetraazatetradecandiimidamin; 1,1'hexamethyl-enebis [5-(2-ethylhexyl)biguanide]) und die Polyhexamethylenbiguanid-Verbindungen des Vantocil® IB Typs (ICI) umfaßt. Darüber hinaus können die Biguanide auch aus den pharmakologisch akzeptabelen Biguaniden der in US 2,684,924, US 2,990,425, US 3,468,898, US 4,022,834, US 4,053,636, US 4,198,392, US 4,891,423, US 5,182,101, US 6,503,952, GB 705,838 und GB 702,268 beschriebenen Verbindungen ausgewählt sein. In besonders bevorzugten Ausführungsformen sind die Biguanide in Form ihrer wasserlöslichen, physiologisch akzeptablen Salze in den Liposomen inkorporiert. Zum Beispiel sind Polyhexamethylenbiguanidhydrochlorid, Chlorhexidindigluconat, Chlorhexidindiacetat, Chlorhexidindihydrochlorid und Alexidinhydrochlorid ganz besonders bevorzugte Biguanide, die in den Liposomen inkorporiert sein können.

Die erfindungsgemäßen Liposomen können zur Herstellung von Wundauflagen in nahezu beliebiger Erscheinungsform verwendet werden, da die antimikrobiellen Eigenschaften der biguanidhaltigen Liposomen überraschenderweise auch nach deren Gefriertrocknung erhalten bleiben.

Überraschenderweise hat sich herausgestellt, daß Polyhexanid in Liposomen stabil inkorporiert werden kann, wenn die Liposomen im wesentlichen frei von Lipiden mit anionischen Kopfgruppen sind.

Liposomen können entweder durch Beladung der Lipidschicht oder durch Beladung der intraliposomalen wäßrigen Phase mit Wirkstoffen beladen werden. Da Polyhexanid eine wasserlösliche Substanz ist, wurde davon ausgegangen, daß diese Moleküle nicht stabil in die Lipid-Doppelschicht von Liposomen inkorporiert werden können, sondern in der intraliposomalen Phase verbleiben sollten. Daher wurde versucht, die intraliposomale wäßrige Phase mit Polyhexanid zu beladen.

Dazu wurden Liposomen unterschiedlicher Zusammensetzung in einem geeigneten Beladungspuffer, vorzugsweise Tris-HCl (pH 7,5) und HEPES (pH 7,0) hergestellt. Zur Herstellung der Liposomen wurden die jeweils verwendeten Lipide in 96 % Ethanol gelöst und mittels des Crossflow-Injektionsverfahrens in die wäßrige Phase druckgesteuert injiziert. Die Größe der dabei entstehenden Liposomen kann durch die lokale Lipidkonzentration am Injektionspunkt eingestellt werden, die durch die Lipidkonzentration in der ethanolischen Phase, der Ethanolkonzentration, dem Injektionsdruck, der Injektionsbohrung und der Fließgeschwindigkeit der wäßrigen Phase am Injektionspunkt bestimmt wird. Unmittelbar nach Injektion der Lipide in die wäßrige Phase wurde die Suspension mit einer weiteren Menge der wäßrigen Phase verdünnt, um die Ethanolkonzentration auf ein tolerierbares Niveau, vorzugsweise auf 7,5 bis 15 %, zu reduzieren.

Als Vorversuch wurden zunächst 2 Liposomensuspensionen hergestellt. Suspension #1 bestand aus hydriertem Soja-Phosphatidylcholin (S100-3 = 87 % Distearylphosphatidylcholin (DSPC) und 13 % Dipalmitoylphosphatidylcholin (DPPC; 10 µmol/ml)) sowie Cholesterol (2 µmol/ml). Suspension #2 bestand aus hydriertem Soja-Phosphatidylcholin (5 µmol/ml), Ei-Phosphatidylglycerol (E-PG; 5 µmol/ml) und Cholesterol (2 µmol/ml). Beide Liposomensuspensionen wurden in Bezug auf Vesikelgröße und Größenverteilung mittels dynamischer Lichtstreuung untersucht und zeigten mit einer durchschnittlichen Vesikelgröße von 120 bis 130 nm Durchmesser und einem Polydispersitätsindex (PDI), der als Maß für die Streuung dient, von 0,23 bis 0,24 vergleichbare Präparatioasergebnisse.

Anschließend wurde diesen Liposomensuspensionen Cosmocil® CQ (Arch Chemicals, Inc, US) zugesetzt (9 Volumenteile Liposomensuspension + 1 Volumenteil Cosmocil® GQ), bei der es sich um eine 20 %-ige, wäßrige Polyhexanid-Lösung (Poly(iminimidocarbonyl)iminohexamethylenhydrochlorid-Lösung) handelt, so daß die Liposomensuspension 2% Polyhexanid enthält. Innerhalb weniger Minuten war bei Suspension #2 eine starke Sedimentation zu beobachten, nicht jedoch bei Suspension #1. Vergleichende Lichtstreumessungen zeigten keine Veränderung der Liposomengröße bei Suspension #1. Bei Suspension #2 mit Polyhexanid zeigte die Vergleichsmessung mit einer durchschnittlichen Vesikelgröße von > 4.000 bis 5.000 nm eine deutliche Veränderung gegenüber der Liposomengröße in Suspension #2 ohne Zusatz von Polyhexanid.

Auch die Präparation von Liposomen mit den gleichen Lipidzusammensetzungen wie bei Suspension #1 und Suspension #2 in Gegenwart von Polyhexanid führte zu einem vergleichbaren Ergebnis. Für die Präparation dieser Liposomen wurde Cosmocil® GQ 1:1 mit PBS verdünnt, um eine 10 %-ige Polyhexanid-Lösung zu bereiten. Diese Lösung wurde mittels Crossflow-Injektionsverfahren bei 55°C in die Lipid/Ethanol-Lösung injiziert und anschließend 1:5 mit PBS verdünnt. Suspension #3 (S 100-3, Cholesterol und Polyhexanid) enthielt Liposomen mit einer durchschnittlichen Größe von 360 bis 370 nm, wie die Lichtstreumessung zeigte. Suspension #4 (S 100-3, E-PG, Cholesterol und Polyhexanid) hingegen zeigte ein ähnliches Sedimentationsverhalten wie Suspension #2 nach Zugabe von Polyhexanid. Die Vesikelgröße in Suspension #4 lag bei 3.000 - 4.000 nm.

Diese Ergebnisse zeigen, daß Liposomen in Gegenwart von Polyhexanid hergestellt werden können, sofern kein Phosphatidylglycerol in die Liposomenmembran inkorporiert wird. Phosphatidylglycerol ist ein Phospholipid mit einer anionischen Kopfgruppe.

Des weiteren konnte bei diesen Versuchen beobachtet werden, daß sich die PBS/Polyhexanid-Lösungen während des Temperierungsprozesses wiederholt leicht trübten. Daher scheint PBS nicht der optimale Puffer für die Herstellung von Polyhexanid-haltigen Liposomen zu sein, auch wenn PBS grundsätzlich als Puffer für die wäßrige Phase geeignet ist.

Anstelle von PBS als Puffer wurden verschiedene Puffer für Vorversuche ausgewählt, wobei darauf geachtet wurde, daß der Puffer bei etwa pH 7,0 eingesetzt werden kann, da die für die Herstellung der Liposomen in Betracht gezogenen Lipide bei diesem pH-Wert eine gute Stabilität aufweisen. Bei diesen Versuchen zeigte sich, daß 50 mM Zitronensäure (pH 7,0) bei Zugabe von Polyhexanid zu einer starken Trübungsreaktion führte, die bei der Temperierung auf die Prozeßtemperatur von 40 bis 50°C zwar verschwand, aber bei Abkühlung wieder auftrat. Als Pufferlösungen, die sich für die Verdünnung von Polyhexanid im Rahmen der Herstellung von Polyhexanid-haltigen Liposomen als besonders geeignet herausstellten, sind beispielsweise 20 mM Tris-Puffer (pH 7,5) und 20 mM HEPES-Puffer (pH 7,0) zu nennen, die beide in Gegenwart von Polyhexanid zu keiner Trübungsreaktion führten. Darüber hinaus führte die Verwendung der beiden letztgenannten Puffer zu Polyhexanid-haltigen Liposomen, die sich hinsichtlich ihrer Größe, Beladung mit Polyhexanid und Stabilität nicht voneinander unterschieden.

In einer weiteren Serie von Versuchen wurden unterschiedliche Lipide verwendet, um Polyhexanid-haltige Liposomen herzustellen, nämlich
- S 100-3 = hydriertes Soja-Phosphatidyloholin (gemischtkettiges, hydriertes Phospholipid aus 87 % Distearylphosphatidylcholin (DSPC) und 13 % Dipalmitoylphosphatidylcholin (DPPC));
- E-PC = Phosphatidylcholin aus Ei (natürliches, gemischtkettiges Phospholipid mit ungesättigten Fettsäuren);
- E80 S = Mischung aus 80 % Phosphatidylcholin (E-PC) und 20 % Phosphatidylethanolamin (E-PE; 1-Palmitoyl-2-oleoyl-sn-glyaero-3-phosphoethanolamin) aus Ei (natürliches, gemischtkettiges Phospholipid mit ungesättigen Fettsäuren und einem Ladungsanteil);
- DMPC = Dimyristoylphosphatidylcholin; und
- DPPC = Dipalmitoylphosphatidylcholin,

Die Größenverteilung der Liposomen zeigte ein heterogenes Ergebnis- Liposomen, die mit S 100-3 bei 55°C mit 10 % Polyhexanid hergestellt wurden, zeigten in Abwesenheit von Cholesterol (Suspension #6) eine monomodale Verteilung mit einer durchschnittlichen Liposomengröße von 200 - 250 nm. Die Anwesenheit von Cholesterol (Suspension #5) führte dagegen zu einer multimodal verteilten Liposomensuspension, die nicht für weitere Untersuchungen verwendet wurde. Bei Polyhexanid-haltigen Liposomen, die mit E-PC bei 35°C hergestellt worden waren, wurde in Anwesenheit von Cholesterol (Suspension #7) eine homogene monomodale Vesikelpopulation mit einem Durchmesser von durchschnittlich 200 nm erhalten, nicht jedoch bei Abwesenheit von Cholesterol (Suspension #8). Bei Suspensionen, die mit E 80-S hergestellt wurden, hatte die An- bzw. Abwesenheit von 15 bis 20 Mol-% Cholesterol (Suspensionen #9 und #10) keinen Einfluß auf die Liposomenbildung. Cholesterol dürfte sogar in Mengen bis zu oder knapp unter 50 Mol-% einsetzbar sein, ohne die Liposomenbildung zu beeinträchtigen, denn Cholesterol ist kein membranbildendes Lipid.

Die monomodale Größenverteilung der Liposomen in den Suspensionen #6, #7, #9 und #10 lassen auf eine geringe oder keine Interaktion von Polyhexanid mit Membranlipiden schließen. Bei den multimodalen Größenverteilungen dürfte das Polyhexanid während des Präparationsprozesses mit der Membran oder einzelnen ihrer Bestandteile interagieren und so eine homogene Vesikelbildung stören.

Für weitere Analysen und Präparationen wurden nur Liposomensuspensionen mit monomodaler Größenverteilung der Vesikel verwendet. Diese Suspensionen wurde in einer 10 ml Amicon-Rührzelle mit einer Celluloseacetat-Membran, welche eine Ausschlußgrenze von 100 kDa hatte, filtriert, um den Anteil nicht inkorporierten Polyhexanids zu entfernen. Anschließend wurde der Polyhexanid-Gehalt aller im Rahmen der Filtration erhaltenen Fraktionen unter Verwendung der Eosin-Testmethode bestimmt. Bei den untersuchten Liposomen waren durchschnittlich 15 bis 25 % des zugesetzten Polyhexanids inkorporiert worden. Die Analyse der Filtrate zeigte auch, daß Polyhexanid stabil in den Liposomen verbleibt, das die Menge Polyhexanid in den aufeinanderfolgenden Filtraten kontinuierlich abnahm und sich der Polyhexanid-Gehalt im Retentat im erwarteten Bereich bewegte.

Entsprechende Versuche wurden auch mit den synthetischen Lipiden DPPC (Dipalmitoylphosphatidylcholin) und DMPC (Dimyristoylphosphatidylcholin) durchgeführt. Dabei wurde gefunden, daß mit DPPC die bei 50 bis 55°C hergestellten Liposomensuspensionen eine multimodale Größenverteilung aufwiesen, unabhängig von der Anwesenheit von Cholesterol (Suspension #11) bzw. Abwesenheit von Cholesterol (Suspension #12). Liposomen aus DMPC und Cholesterol (Suspension #13), die wie auch die Liposomen aus ungesättigten Lipidmischungen (Suspensionen #7 und #8) bei 35°C hergestellt wurden, zeigten mit einem durchschnittlichen Durchmesser der Liposomen von etwa 400 - 500 nm und einer stabilen Inkorporation von etwa 30 - 40 % des zugesetzten PHMB bessere Ergebnisse.

Da nicht zweifelsfrei geklärt werden konnte, ob der für die vorgenannten Versuche verwendete Tris-Puffer für pharmazeutische Formulierungen oder Medizinprodukte eingesetzt werden kann, wurden die Liposomenpräparationen, die unter Verwendung von Tris-Puffer ein zufriedenstellendes Ergebnis erbracht hatten, nämlich Suspensionen #7, #9, #10 und #13, unter Verwendung von 20 mM HEPES-Puffer (pH 7,0) anstelle von Tris-Puffer wiederholt, denn HEPES-Puffer werden nachgewiesenermaßen in pharmazeutischen Formulierungen verwendet.

Es konnten keine Unterschiede zwischen den mit Tris-Puffer und HEPES-Puffer hergestellten Liposomen festgestellt werden, wie der Vergleich der Versikelgrößen und der Polyhexanid-Beladung der Suspensionen #7, #9, #10 und #13 mit denen der Suspensionen #15 bis #18 zeigt (siehe Tabelle 1). Sowohl hinsichtlich der Vesikelgröße und ihrer Größenverteilung, als auch im Eiaschlußverhalten von Polyhexanid waren keine signifikanten Unterschiede nachzuweisen. Lediglich die E-PC/Cholesterol-haltige Liposomensuspensionen (Suspensionen #7 und #15) zeigten bei den Analysen eine größere Streuung der Meßwerte als die übrigen Liposomenpräparationen.

In weiteren Experimenten wurde der Lipid/Ethanol-Lösung im Präparationsprozeß noch Vitamin E zugesetzt, um die aus ungesättigten Lipiden hergestellten Liposomen während ihrer Lagerung vor oxidativem Abbau zu schützen und um die Wundheilung durch Zugabe dieses Radikalfängers verbessern zu können.

Zunächst wurde die optimale Menge an Vitamin E bestimmt, die den Vesikelformulierungsprozeß nicht störend beeinflußt. Dabei wurde ermittelt, daß der Lipidphase bis zu 40 Mol-% an Vitamin E zugesetzt werden konnte, ohne daß die Vesikelbildung beeinträchtigt wurde.

Auf diesen Ergebnissen aufbauend wurden in einer weiteren Serie Polyhexanid-haltige Liposomen mit 20 Mol-% Vitamin E hergestellt, anschließend filtriert und analysiert. Die Eigenschaften der aus diesen Präparationen hergestellten Liposomen (Suspensionen #19 bis #21) glichen hinsichtlich Vesikelgröße und Einschlußmengen an Polyhexanid den Eigenschaften der Lioposomen aus den Präparationen ohne Zusatz von Vitamin E (Suspensionen #15, #16, #17). Auch unter Verwendung von Vitamin E wurden etwa 20 bis 25 % des zugesetzten PHMB liposomal inkorporiert.

Die Beladung von Liposomen mit Polyhexanid ist abhängig von ihrer Vesikelgröße. Liposomen mit 150 bis 200 nm Durchmesser können konstant mit 15 bis 20% des zur Präparation zugesetzten Polyhexanids, Liposomen mit 400 bis 500 nm Durchmesser können konstant mit 30 bis 40% des zur Präparation zugesetzten Polyhexanids beladen werden.

Zur Herstellung von antiseptisch wirkenden Liposomensuspensionen können neben dem bevorzugten Polyhexanid, dessen gute Verträglichkeit und breites Wirkuagsspektrum nachgewiesen ist, auch alle anderen Biguanide verwendet werden, die eine antimikrobielle Wirkung besitzen und physiologisch akzeptabel sind.

Die Molekulargewichte, die die zu verwendenden Polyhexanide aufweisen sollten, unterliegen keiner wesentlichen Beschränkung. Polyhexanide aller bislang üblichen Molekulargewichte können verwendet werden. Das bevorzugte PHMB weist ein Molekulargewicht im Bereich von 1.500 bis 15.000 g/mol auf. Bevorzugt werden Polyhexanide mit einem Polymerisationsgrad von 12-16. Der Polymerisationsgrad gibt an, wie viele monomere Moleküle während der Polymerisation durchschnittlich zu einem Makromolekül verbunden werden.

Die Einarbeitung von liposomal inkorporiertem Polyhexanid oder einem anderen liposomal inkorporierten Biguanid in eine Wundauflage kann auf verschiedene Weise erfolgen. Die Polyhexanid-haltigen Liposomen können beispielsweise in eine Polymerlösung des Trägermaterials für die Wundauflage eingearbeitet werden. Durch Abdampfen oder Gefriertrocknen wird anschließend das Lösungsmittel entfernt. Um eine feuchte Wundauflage zu erzeugen, kann das Lösungsmittel ganz oder teilweise in der Wundauflage bzw. dem Trägermaterial für die Wundauflage verbleiben, bevor diese weiterverarbeitet wird.

Alternativ kann man die Suspension biguanidhaltiger Liposomen auch mit Verfahren auf ein Trägermaterial aufbringen, die bei Polyhexanid-Lösungen angewendet werden. So können die biguanidhaltige Liposomen durch Berieseln oder Besprühen auf das Trägermaterial aufgetragen werden.

Die vorliegende Erfindung betrifft somit antiseptische Zubereitungen auf Basis eines in Liposomen eingeschlossenen antimikrobiellen Wirkstoffs, die sich dadurch auszeichnen, daß die Liposomen keine Lipide mit anionischen Kopfgruppen enthalten, in ihrem Inneren ein wäßriges Milieu aufweisen und in dem wäßrigen Milieu mindestens ein antimikrobieller Wirkstoff aus der Gruppe der Biguanide enthalten ist.

**Tabelle 1: Übersicht über die Zusammensetzungen und Eigenschaften der hergestellten Liposomensuspensionen**

| # | Lipidphase | Wäßrige Phase | Größe der Vesikel | Größeavorteilung der Vesikel | Beladung mit PHMB |
|---|---|---|---|---|---|
| 1 | S 100-3 Cholesterol | PBS | 120 - 130 | monomodal | |
| 2 | S 100-3 Cholesterol E-PG | PBS | > 5000 | monomodal | |
| 3 | S 100-3 Cholesterol | PHMB PBS | 360 - 370 | bimodal | |
| 4 | S 100-3 Cholesterol E-PG | PHMB PBS | > 5000 | monomodal | |
| 5 | S 100-3 Cholesterol | PHMB Tris | | multimodal | |
| 6 | S 100-3 | PHMB Tris | 200- 250 | monomodal | 15 - 25 |
| 7 | E-PC Cholesterol | PHMB Tria | 200 | monomodal | 15 - 25 |
| 8 | E-PC | PHMB Tris | | multimodal | |
| 9 | E 80-s Cholesterol | PHMB Tris | 150 - 200 | monomodal | 15 - 25 |
| 10 | E 80-s | PHMB Tris | 150 - 200 | monomodal | 15 - 25 |
| 11 | DPPC Cholesterol | PHMB Tris | | multimodal | |
| 12 | DPPC | PHMB Tris | | multimodal | |
| 13 | DMPC Cholesterol | PHMB Tris | 400 - 500 | monomodal | 30 - 40 |
| 14 | DMPC | PHMB Tris | | | |
| 15 | E-PC Cholesterol | PHMB HERPES | 200 | monomodal | 15 - 25 |
| 16 | E 80-S Cholesterol | PHMB HEPES | 150 - 200 | monomodal | 15 - 25 |
| 17 | E 80-S | PHMB HERPES | 150 - 200 | monomodal | 15 - 25 |
| 18 | DMPC Cholesterol | PHMB HEPES | 400 - 500 | monomodal | 30 - 40 |
| 19 | E-PC Cholesterol Vitamin E | PHMB HEPES | 200 nm | monomodal | 15 - 25 % |
| 20 | E80-S Cholesterol Vitamin E | PHMB HEPES | 150 - 200 m | monomodal | 15 - 20 % |
| 21 | E 80-S Vitamin E | PHMB HEPES | 150 - 200 nm | monomodal | 15 - 25 % |
| 22 | DMPC Vitamin E | PHMB HEPES | 400 - 500 nm | monomodal | 30 - 40 % |

Vorzugsweise umfassen die erfindungsgemäßen Liposomen Phospholipide, die aus der Gruppe der natürlichen und synthetischen Phospholipide ausgewählt sind, die Phosphatidylcholin, Phosphatidylethanolamin, Dimyristoylphosphatidylcholin und deren Mischungen miteinander umfaßt. Die natürlichen Phospholipide stammen vorzugsweise aus Eiern oder Sojabohnen.

Bei Verwendung von synthetischen Phospholipide zur Herstellung der Liposomen können Phospholipide mit Kettenlängen von 14 bis 24 Kohlenstoffatomen verwendet werden. Je langer die Kohlenstoff-Ketten der Phospholipide, desto besser dürfte das Biguanid in den Liposomen verbleiten.

In einer besonders bevorzugten Ausführungsform umfaßt die erfindungsgemäße Zusammensetzung Polyhexanid als lineares polymeres Biguanid mit antimikrobieller Wirkung, wobei Polyhexanide mit einem Molekulargewicht von 1.500 bis 15.000 g/mol und/oder mit einem Polymerisationsgrad von 12 - 16 ganz besonders bevorzugt werden.

Vorzugsweise handelt es sich bei dem wäßrigen Milieu um einen Puffer, wobei PBS, Tris-Puffer und HEPES-Puffer ganz besonders bevorzugt werden. Das wäßrige Milieu sollte einen pH-Wert von 6 bis 8 aufweisen, vorzugsweise liegt der pH-Wert bei 7,0 bis 7,5.

In einer bevorzugten Ausführungsform enthalten die Liposomen, genauer gesagt die Lipid-Doppelschichten der Liposomen, Cholesterol.

Der Cholesterol-Gehalt in den Liposomen kann bis zu 50 Mol-% betragen, vorzugsweise liegt der Cholesterol-Gehalt bei 15 bis 20 Mol-%.

In einer weiteren bevorzugten Ausführungsform enthalten die Liposomen, genauer gesagt die Lipid-Doppelschichten der Liposomen, gegebenenfalls zusätzlich zu dem Cholesterol, Vitamin E in einer Menge von bis zu 40 Mol-%, vorzugsweise eine Menge von 20 Mol-%, jeweils bezogen auf die Gesamtlipide.

Die erfindungsgemäßen Liposomen weisen vorzugsweise eine mittlere Größe von 50 bis 800 nm auf, besonders bevorzugt werden Liposomen mit einer mittleren Größe von 150 bis 500 nm.

Die erfindungsgemäße Zubereitung kann beispielsweise in Form einer Suspension, Emulsion, Lotion, Tinktur, eines Sprays, Gels, einer Creme oder Salbe vorliegen.

Die vorliegende Erfindung betrifft auch Verfahren zur Herstellung antiseptischer Zusammensetzungen auf Basis eines in Liposomen eingeschlossenen antimikrobiellen Wirkstoffs aus der Gruppe der Biguanide, wobei die Liposomen keine Lipide mit anionischen Kopfgruppen enthalten und sich das Verfahren dadurch auszeichnet, daß zunächst eine ethanolische Lipidphase in eine wäßrige, den antimikrobiellen Wirkstoff aus der Gruppe der Biguanide enthaltende Phase druckgesteuert injiziert, die wäßrige Phase nach erfolgter Vesikelbildung mit einem Puffer verdünnt, und nicht inkorporierter Wirkstoff anschließend entfernt wird. Vorzugsweise wird der Puffer zur Verdünnung der Liposomensuspension verwendet, der zur Bereitung der wäßrigen Phase verwendet wurde.

Vorzugsweise werden bei dem erfindungsgemäßen Verfahren für die Lipidphase Phospholipide der Gruppe der natürlichen oder synthetischen Phospholipide verwendet, die aus der Gruppe ausgewählt sind, die aus Phosphatidylcholin, Phosphatidylethanolamin, Dimyristoylphosphatidylcholin und deren Mischungen miteinander besteht, wobei die natürlichen Phospholipide vorzugsweise aus Eiern oder Sojabohnen stammen.

Als bevorzugtes Biguanid mit antimikrobieller Wirkung werden vorzugsweise Chlorhexidin, Fluorhexidin, Alexidin oder Polyhexanide verwendet, besonders bevorzugt Polyhexanide mit einem Molekulargewicht von 1.500 bis 15.000 g/mol und/oder mit einem Polymerisationsgrad von 12 bis 16.

Die wäßrige Phase wird bei dem erfindungsgemäßen Verfahren vorzugsweise aus einem Puffer-System gebildet, besonders bevorzugt aus der Gruppe, die aus PBS (Phosphat-gepufferte Kochsalzlösung), Tris-Puffer und HEPES-Puffer besteht. Der pH der wäßrigen Phase wird auf einen Wert von 6 bis 8 eingestellt, besonders bevorzugt liegt der pH-Wert bei 7,0 bis 7,5.

In einem besonders bevorzugten Verfahren enthält die Lipidphase Cholesterol in einer Menge von 0 bis 50 Mol-%, vorzugsweise 15 bis 20 Mol-% und/oder Vitamin E in einer Menge von 0 bis 40 Mol-%, vorzugsweise von 20 Mol-%, jeweils gezogen auf die Gesamtlipide.

Die vorliegende Erfindung betrifft auch die Verwendung der erfindungsgemäßen antiseptischen Zusammensetzung, insbesondere die Verwendung zur Herstellung von Wundauflagen, deren Trägermaterial beispielsweise durch Berieseln, Besprühen oder Imprägnieren mit den antimikrobiellen Liposomen versehen werden können.

Als Trägermaterialien für die Herstellung der wundauflagen kommen alle dafür gebräuchlichen und dem Fachmann geläufigen Materialien in Betracht, z. B. Kollagen, Cellulosen und Cellulosederivaten, Polyurethane, Alginate, allein oder in Kombination mit Polysacchariden aus der Gruppe, die aus Alginaten, Hyaluronsäure und ihren Salzen (Hyaluronaten), Pektinen, Carrageenanen, Xanthanen, sulfatierten Dextranen, Cellulosederivaten, oxidierter Cellulose wie oxidierter regenerierter Cellulose, Chondroitin, Chondroitin-4-sulfat, Chondroitin-6-sulfat, Heparin, Heparansulfat, Keratansulfat, Dermatansulfat, Stärkederivaten und deren Mischungen besteht.

In einer anderen bevorzugten Verwendung wird eine Polymerlösung, z. B. eine Kollagenlösung, mit der Liposomensuspension vermischt und anschließend das bzw. die Lösungsmittel durch Trocknen oder Gefriertrocknen ganz oder teilweise entfernt, so daß man Schwämme erhalten kann, die mit den antimikrobiell wirksamen Liposomen versehen sind.

Die vorliegende Erfindung bezieht sich daher auch auf Wundauflage, die biguanidhaltige Liposomen umfaßt und beispielsweise auf Cellulose, einem Cellulosederivat wie Carboxymethylcellulosen, Alginaten, Chitosan, Stärke oder Stärkederivaten, Kollagen, Polyacrylaten, Polyurethan oder Mischungen der vorgenannten Verbindungen als Trägermaterial basiert.

Die bevorzugten Ausführungsformen der erfindungsgemäßen Wundauflage sind Hydrogele, Hydrocolloide, Schwämme, Folien, Membranen, Vliese, Gewebe, Gewirke, andere textile Flächengebilde, Kardenbänder, Tamponaden und dergleichen.

Ganz besonders bevorzugt enthalten die erfindungsgemäßen Wundauflagen das liposomal inkorporierte, antimikrobiell wirksame Biguanid, vorzugsweise Polyhexanid, in einer Menge von 0,01 bis 1,0 Gew.-%, bezogen auf das Trockengewicht der Auflage.

### Ausführungsbeispiel

Zur Herstellung von antiseptisch ausgerüsteten Kollagenschwämmen wurden 1%-ige Kollagensuspensionen (bovinen Urprungs) mit definierten Mengen einer Liposomensuspension (in Tris-Puffer) intensiv durchmischt und anschließend in eine Kunststoffschale gegeben. Die Mischung wurde dann bei -50°C tiefgefroren und anschließend lyophilisiert. Auf diese Weise wurden Kollagenschwämme mit einem Polyhexanid-Gehalt von 0,05 Gew.-%, 0,1 Gew.-%, 0,5 Gew.-% oder 1 Gew.-%, bezogen auf das Trockengewicht des Kollagens, hergestellt.

Es wurden drei unterschiedliche Liposomensuspensionen verwendet:
I. Liposomen aus E 80-S, Cholesterol und Vitamin. E (Suspension #20);
II. Liposomen aus E 80-S und Vitamin E (Suspension #21); und
III. Liposomen aus DMPC und Cholesterol (Suspension #22).

Die antimikrobielle Wirkung der Polyhexanid-haltige Liposomen umfassenden Kollagenschwämme sowie der für die Herstellung dieser Kollagenschwämme verwendeten Liposomensuspensionen auf *Staphylococcus aureus*, *Pseudomonas aerugiaosa* und Candida *albicans* wurde mittels Agar-Diffusions-Tests näher untersucht. Zu diesem Zweck wurden die Mikroorganismen für 24 h bei 30 bis 35°C in einem nicht selektiven Flüssigmedium kultiviert und anschließend mit einer 1 %-igen NaCl-Lösung, die 1 % Pepton enthielt, auf 1x10⁸ CFU/ml (colony forming units) bzw. 3,8x10⁷ CFU/ml (*C. albicans*) verdünnt. Jeweils 100 µl der Verdünnungen wurden auf CSA- bzw. SDA-Platten (CSA = Casein-Soja-Pepton-Agar; SDA = Sabouraud-Dextrose-Agar) ausgestrichen. Die Agar-Platten wurden 3 bis 5 Minuten getrocknet.

Nach Aufbringen der Lösungen, Liposomensuspensionen oder Kollagenschwämme wurden die Platten für 24 h bei 30 bis 35°C bzw. 48 h bei 20 bis 25°C (*C. albicans*) inkubiert, bevor der Bereich der Hemmung ermittelt wurde. Der Bereich der Hemmung wurde quantifiziert, indem der Abstand vom Schwämmchen bzw. dem Loch in der Agarplatte für die einzufüllende Lösung oder Suspension zum Rand des Hemmhofs gemessen wird. Die Ergebnisse sind in semiquantitativer Form in Tabelle 2 zusammengefaßt.

Die Kollagenschwämme, die mit Polyhexanid beladene Liposomen umfaßten, zeigten eine gute antimikrobielle Wirkung gegenüber den drei untersuchten Mikroorganismen (*S. aureus*, *P. aeruginosa* und *C. albicans*). Bei diesen Untersuchungen zeigten die Kollagenschwämme mit Polyhexanid-haltigen Liposomen auf Basis von DMPC die schwächste mikrobiozide Wirkung gegenüber den untersuchten Mikroorganismen, verglichen mit den Kollagenschwämmen, die mit Polyhexanid-haltigen Liposomen auf Basis von E 80-S versehen waren. Die Kollagenauflagen mit Polyhexanid-haltigen Liposomen auf Basis von E 80-S zeigten bereits bei Beladung mit geringen Polyhexanid-Mengen eine gute Wirkungen gegenüber *S. aureus* und C. *albicans.* Die mikrobiozide Wirkung dieser Wundauflagen gegenüber *P. aeruginosa* war uneinheitlich, allerdings kam es auch bei den Wundauflagen mit niedriger Polyhexanid-Beladung zu keiner Verkeimung der wundauflage.

Eine Gefriertrocknung der Liposomensuspension hatte keine negativen Einflüsse auf die antimikrobielle Wirkung der Präparationen.

Die vorliegenden Ergebnisse zeigen, daß sich mit liposomal inkorporiertem Polyhexanid Wundauflagen, zumindest auf Basis von oder mit Kollagen, herstellen lassen, die eine ausgezeichnete antimikrobielle Wirkung aufweisen.

**Tabelle 2: Semiquantitative Auswertung der antimikrobiellen Wirkung von Polyhexanid-haltigen Zubereitungen**

| | Zusammensetzung | Art der Zubereitung | PHMB-Gehalt | *S. aureus* | *P*. *aeruginosa* | *C. albicans* |
|---|---|---|---|---|---|---|
| A | Liposomen aus | Kollagen- | 1,0 | +++ | + | +++ |
| | E 80-S | schwamm | 0,5 | +++ | + | +++ |
| | Cholesterol | | 0.1 | ++ | + | +++ |
| | Vitamin E | | 0,05 | ++ | + | +++ |
| B | Liposomen aus | Kollagen- | 1,0 0 | +++ | +++ | +++ |
| | E 80-S | schlamm | 0,5 | +++ | +++ | +++ |
| | Vitamin E | | 0,1 | +++ | + | +++ |
| | | | 0,05 | +++ | + | ++ |
| C | Liposomen aus | Kollagen- | 1,0 | ○ | ○ | ○ |
| | DMPC | schlamm | 0,5 | ○ | ○ | ○ |
| | Cholesterol | | 0,1 | ○ | ○ | ○ |
| | | | 0,05 | ○ | ○ | ○ |
| D | Liposomen aus | Suspension | 1,0 | +++ | + | +++ |
| | E 80-S | | 0,5 | +++ | + | +++ |
| | Cholesterol | | 0,1 | ++ | + | +++ |
| | Vitamin E | | 0,05 | ++ | + | + |
| E | Liposomen aus | Suspension | 1,0 | +++ | + | +++ |
| | B 80-S | | 0,5 | +++ | + | +++ |
| | Vitamin E | | 0,1 | +++ | + | +++ |
| | | | 0,05 | +++ | + | + |
| P | Liposomen aus | Suspension | 1,0 | +++ | + | + |
| | DMPC | | 0,5 | +++ | + | + |
| | Cholesterol | | 0,1 | +++ | + | + |
| | | | 0,05 | +++ | + | + |
| H | Lötung aus PBMB | Kollagen- | 1,0 | +++ | + | + |
| | | schwamm | 0,5 | +++ | + | + |
| | | | 0,1 | ++ | + | ○ |
| | | | 0,05 | ++ | + | ○ |
| I | Liposomen aus | Suspension, | 1,0 | +++ | +++ | +++ |
| | E 80-S | lyophilisiert | 0,5 | +++ | +++ | +++ |
| | Vitamin B | | 0,1 | +++ | ++ | +++ |
| | | | 0,05 | +++ | + | +++ |
| J | Liposomen aus | Suspension | 1,0 | +++ | ++ | +++ |
| | E 80-S | lyophilisiert | 0,5 | +++ | ++ | +++ |
| | Cholesterol | | 0,1 | +++ | + | +++ |
| | Vitamin B | | 0,05 | +++ | + | +++ |
| K | Liposomen aus | Suspension | 1,0 | +++ | + | + |
| | DMPC | lyophilisiert | 0,5 | +++ | + | + |
| | Vitamin B | | 0,1 | ++ | + | + |
| | | | 0,05 | + | + | + |
| L | PBMB | Lösung | 1,0 | +++ | +++ | +++ |
| | | | 0,5 | +++ | +++ | +++ |
| | | | 0,1 | +++ | +++ | +++ |
| | | | 0,05 | +++ | +++ | +++ |
| M | | Kollagenschwamm | 0,0 | - | - | - |

## Patentansprüche

1. Antiseptische Zuaammensetzung auf Basis eines in Liposomen eingeschlossenen antimikrobiellen Wirkstoffs, **dadurch gekennzeichnet, daß** die Liposomen keine Lipide mit anionischen Kopfgruppen enthalten, und die Liposomen in ihrem Inneren ein wäßriges Milieu aufweisen in dem Polyhexanid als antimikrobieller Wirkstoff enthalten ist.

2. Antiseptische Zusammensetzung nach Anspruch 1, **dadurch gekennzeichnet, daß** die Phospholipide aus der Gruppe der natürlichen und synthetischen Phospholipide ausgewählt sind, die Phosphatidylcholin, Phosphatidylethanolamin, Dimyristoylphosphatidylcholin und deren Mischungen miteinander umfaßt.

3. Antispetische Zusammensetzung nach Anspruch 2, **dadurch gekennzeichnet, daß** die Liposomen Phospholipide mit einer Acylkettenlänge von mindestens 14 Kohlenstoffatomen, vorzugsweise von mindestens 16 Kohlenstoffatomen, enthalten.

4. Antiseptische Zusammensetzung nach Anspruch 2, **dadurch gekennzeichnet, daß** die natürlichen Phospholipide aus Ei oder Sojabohnen stammen.

5. Antiseptische Zubereitung nach einem der vorhergehenden Ansprüche, **dadurch gekennzeichnet, daß** das Polyhexanid ein Molekulargewicht von 1.500 bis 15.000 g/mol und/oder einen Polymerisationsgrad von 12 bis 16 aufweist.

6. Antiseptische Zusammensetzung, nach einem der vorangehenden Ansprüche, **dadurch gekennzeichnet, daß** das wäßrige Milieu ein Puffer ist, der aus der Gruppe ausgewählt ist, die aus PBS, Tris-Puffer und HEPES-Puffer besteht.

7. Antiseptische Zusammensetzung nach einem der vorangehenden Ansprüche, **dadurch gekennzeichnet, daß** der pH-Wert des wäßrigen Milieus bei 6 bis 8, vorzugsweise bei 7,0 bis 7, 5, liegt.

8. Antiseptische Zusammensetzung nach einem der vorangehenden Ansprüche, **dadurch gekennzeichnet, daß** die Liposomen Cholesterol enthalten, vorzugsweise in einer Menge von bis zu 50 Mol-%, besonders bevorzugt in einer Menge von 15 bis 20 Mol-%, jeweils bezogen auf die Gesamtlipide.

9. Antiseptische Zusammensetzung nach einem der vorangehenden Ansprüche, **dadurch gekennzeichnet, daß** die Liposomen Vitamin E enthalten, vorzugsweise in einer Menge von bis zu 40 Mol-%, besonders bevorzugt in einer Menge von 20 Mol-%, jeweils bezogen auf die Gesamtlipide.

10. Antiseptische Zusammensetzung nach einem der vorangehenden Ansprüche, **dadurch gekennzeichnet, daß** die Liposomen eine mittlere Größe von 50 bis 800 nm, vorzugsweise von 150 bis 500 nm, aufweisen.

11. Antiseptische Zusammensetzung nach einem der vorangehenden Ansprüche, **dadurch gekennzeichnet, daß** sie in Form einer Suspension, Emulzion, Lotion, Tinktur, eines Sprays, Gels, einer Creme oder Salbe vorliegt.

12. Verfahren zur Herstellung einer antiseptischen Zusammensetzung auf der Basis eines in Liposomen eingeschlossenen antimikrobiellen Wirkstoffs, wobei die Liposomen keine Lipide mit anionischen Kopfgruppen enthalten, und die Liposomen in ihrem inneren ein wäßriges Milieu aufweisen in dem Polyhexanid als antimikrobieller Wirkstoff enthalten ist, **dadurch gekennzeichnet, daß**
- eine ethanolische Lipidphase in eine wäßrige, den antimikrobiellen Wirkstoff aus der Gruppe der Biguanide enthaltende Phase druckgesteuert injiziert,
- die wäßrige Phase nach erfolgter Vesikelbildung mit einem Puffer verdünnt, und
- nicht inkorporierter wirkstoff entfernt wird.

13. Verfahren nach Anspruch 12, **dadurch gekennzeichnet, daß** die Lipidphase Phospholipide umfaßt, die aus der Gruppe der natürlichen oder synthetischen Phospholipide ausgewählt werden, vorzugsweise aus der Gruppe, die aus Phosphatidylcholin, Phosphatidylethanolamin, Dimyristoylphosphatidyloholin und deren Mischungen miteinander besteht.

14. Verfahren nach Anspruch 13, **dadurch gekennzeichnet, daß** die Phospholipide aus der Gruppe von Phospholipiden ausgewählt werden, die eine Acylkettenlänge von mindestens 14 Kohlenstoffatomen, vorzugsweise aus der Gruppe von Phospholipiden, die eine Acylkottenlänge von mindestens 16 Kohlenstoffatomen aufweisen.

15. Verfahren nach Anspruch 13, **dadurch gekennzeichnet, daß** die natürlichen Phospholipide aus Ei oder Sojabohnen stammen.

16. Verfahren nach einem der Ansprüche 12 bis 15, **dadurch gekennzeichnet, daß** das Polyhexanid aus der Gruppe der Polyhexanide ausgewählt wird, die ein Molekulargewicht von 1.500 bis 15.000 g/mol und/oder einen polymerisationsgrad von 12 bis 16 aufweisen.

17. Verfahren nach einem der Ansprüche 12 bis 16, **dadurch gekennzeichnet, daß** das wäßrige Milieu ein Puffer ist, der aus der Gruppe ausgewählt ist, die aus PBS, Tris-Puffer und HEPES-puffer besteht.

18. verfahren nach einem der Ansprüche 12 bis 17, **dadurch gekennzeichnet, daß** der pH-Wert des wäßrigen Milieus bei 6 bis 8, vorzugsweise bei 7,0 bis 7,5, liegt.

19. verfahren nach einem der Ansprüche 12 bis 18, **dadurch gekennzeichnet, daß** die Lipidphase Cholesterol enthält, vorzugsweise in einer Menge von bis zu 50 Mol-%, besonders bevorzugt in einer Menge von 15 bis 20 Mol-%, jeweils bezogen auf die Gesamtlipide.

20. Verfahren nach einem der Ansprüche 12 bis 19, **dadurch gekennzeichnet, daß** die Lipidphase vitamin E enthält, vorzugweise in einer Menge von bis zu 40 Mol-%, besonders bevorzugt in einer Menge von 20 Mol-%, jeweils bezogen auf die Gesamtlipide.

21. Verwendung einer antiseptischen Zusammensetzung nach einem der Ansprüche 1 bis 11 zur Herstellung einer Wundauflage.

22. Verwendung nach Anspruch 21, **dadurch gekennzeichnet, daß** das Trägermaterial der Wundauflage mit der Zusammensetzung berieselt, besprüht oder imprägniert wird.

23. Verwendung nach Anspruch 22, **dadurch gekennzeichnet, daß** eine Polymerlösung mit der Zusammensetzung vermischt und durch Trocknen oder Gefriertrocknen das Lösungsmittel ganz oder teilweise entzogen wird.

24. Wundauflage, **dadurch gekennzeichnet, daß** sie eine antiseptische Zusammensetzung gemäß einem der Ansprüche 1 bis 11 umfaßt.

25. Wundauflage gemäß Anspruch 24, **dadurch gekennzeichnet, daß** sie auf Cellulose, Cellulosederivaten wie Carboxymethylcellulosen, Alginaten, Chitosan, Stärke, Kollagen, Polyacrylaten, Polyurethan oder Mischungen der vorgenannten Verbindungen als Trägermaterial basiert.

26. Wundauflage gemäß Anspruch 24 oder 25, **dadurch gekennzeichnet, daß** sie in Form eines Hydrogels, Hydroeolloids, Schwamms, einer Folie, Membran, eines Vlieses, Gewebes, Gewirkes, Kardenbandes oder einer Tamponade vorliegt.

27. Wundauflage nach einem der Ansprüche 24 bis 26, **dadurch gekennzeichnet, daß** die Wundauflage das Biguanid zeine Menge von 0,01 bis 1,0 Gew.-%, bezogen auf das Trockengewicht der Auflage, enthält.

## Claims

1. Antiseptic composition based on an antimicrobial active agent enclosed in liposomes, **characterized in that** said liposomes are free of lipids with anionic head groups, and that said liposomes have an aqueous medium in their interior which contains polyhexanide as antimicrobial active agent.

2. Antiseptic composition according to claim 1, **characterised in that** the phospholipids are selected from the group of the natural and synthetic phospholipids which comprises phosphatidylcholine, phosphatidylethanolamine, dimyristoylphosphatidylcholine, and mixtures thereof.

3. Antiseptic composition according to claim 2, **characterised in that** the liposomes contain phospholipids with an acyl chain length of at least 14 carbon atoms, preferably of at least 16 carbon atoms.

4. Antiseptic composition according to claim 2, **characterised in that** the natural phospholipids originate from eggs or soya beans.

5. Antiseptic preparation according to any one of the preceding claims, **characterised in that** the polyhexanide has a molecular weight of from 1,500 to 15,000 g/mol and/or a degree of polymerisation of from 12 to 16.

6. Antiseptic composition according to any one of the preceding claims, **characterised in that** the aqueous medium is a buffer which is selected from the group consisting of PBS, Tris buffer and HEPES buffer.

7. Antiseptic composition according to any one of the preceding claims, **characterised in that** the pH value of the aqueous medium is from 6 to 8, preferably 7.0 to 7.5.

8. Antiseptic composition according to any one of the preceding claims, **characterised in that** the liposomes contain cholesterol, preferably in an amount of up to 50 mol %, more preferably in an amount of from 15 to 20 mol %, in each case relative to the total lipids.

9. Antiseptic composition according to any one of the preceding claims, **characterised in that** the liposomes contain vitamin E, preferably in an amount of up to 40 mol %, more preferably in an amount of 20 mol %, in each case relative to the total lipids.

10. Antiseptic composition according to any one of the preceding claims, **characterised in that** the liposomes have a mean size of from 50 to 800 nm, preferably from 150 to 500 nm.

11. Antiseptic composition according to any one of the preceding claims, **characterised in that** it is present in the form of a suspension, emulsion, lotion, tincture, spray, gel, cream or ointment.

12. Method for the production of an antiseptic composition based on an antimicrobial active agent enclosed in liposomes, wherein said liposomes are free of lipids with anionic head groups and wherein said liposomes have an aqueous medium in their interior which contains polyhexanide as antimicrobial active agent, **characterised in that**
- an ethanolic lipid phase is injected, by pressure-controlled injection, into an aqueous phase containing the antimicrobial active agent from the group of the biguanides,
- after the formation of vesicles has taken place, the aqueous phase is diluted with a buffer, and
- unincorporated active agent is removed.

13. Method according to claim 12, **characterised in that** the lipid phase comprises phospholipids which are selected from the group of the natural or synthetic phospholipids, preferably from the group which consists of phosphatidylcholine, phosphatidylethanolamine, dimyristoylphosphatidylcholine, and mixtures thereof.

14. Method according to claim 13, **characterised in that** the phospholipids are selected from the group of the phospholipids which have an acyl chain length of at least 14 carbon atoms, and preferably from the group of phospholipids which have an acyl chain length of at least 16 carbon atoms.

15. Method according to claim 13, **characterised in that** the natural phospholipids originate from eggs or soya beans.

16. Method according to any one of claims 12 to 15, **characterised in that** the polyhexanide is selected from the group of the polyhexanides which have a molecular weight of from 1,500 to 15,000 g/mol and/or a degree of polymerisation of from 12 to 16.

17. Method according to any one of claims 12 to 16, **characterised in that** the aqueous medium is a buffer which is selected from the group consisting of PBS, Tris buffer and HEPES buffer.

18. Method according to any one of claims 12 to 17, **characterised in that** the pH value of the aqueous medium is from 6 to 8, preferably 7.0 to 7.5.

19. Method according to any one of claims 12 to 18, **characterised in that** the lipid phase contains cholesterol, preferably in an amount of up to 50 mol %, more preferably in an amount of from 15 to 20 mol %, in each case relative to the total lipids.

20. Method according to any one of claims 12 to 19, **characterised in that** the lipid phase contains vitamin E, preferably in an amount of up to 40 mol %, more preferably in an amount of 20 mol %, in each case relative to the total lipids.

21. Use of an antiseptic composition according to any one of claims 1 to 11 for the production of a wound dressing.

22. Use according to claim 21, **characterised in that** the carrier material of the wound dressing is sprinkled, sprayed or impregnated with the composition.

23. Use according to claim 22, **characterised in that** a polymer solution is mixed with the composition and that the solvent is completely or partially withdrawn by drying or freeze drying.

24. Wound dressing, **characterised in that** it comprises an antiseptic composition according to any one of claims 1 to 11.

25. Wound dressing according to claim 24, **characterised in that** it is based on cellulose, cellulose derivatives, such as carboxymethyl celluloses, alginates, chitosan, starch, collagen, polyacrylates, polyurethane, or mixtures of the afore-mentioned compounds, as carrier material.

26. Wound dressing according to claim 24 or 25, **characterised in that** it is present in the form of a hydrogel, a hydrocolloid, a sponge, a film, a membrane, a nonwoven fabric, a woven fabric, a knit fabric, card slivers, or a tamponade.

27. Wound dressing according to any one of claims 24 to 26, **characterised in that** said wound dressing contains the biguanide in an amount of from 0.01 to 1.0 wt %., relative to the dry weight of the dressing.

## Revendications

1. Composition antiseptique à base d'une substance active antimicrobienne incorporée dans des liposomes, **caractérisée en ce que** les liposomes ne contiennent aucun lipide comprenant des groupes de tête anioniques, et l'intérieur des liposomes présente un milieu aqueux dans lequel est contenu du polyhexanide à titre de substance active antimicrobienne.

2. Composition antiseptique selon la revendication 1, **caractérisée en ce que** les phospholipides sont choisis parmi le groupe des phospholipides naturels et synthétiques, qui comprend la phosphatidylcholine, la phosphatidyl-éthanolamine, la dimyristoylphosphatidylcholine et leurs mélanges réciproques.

3. Composition antiseptique selon la revendication 2, **caractérisée en ce que** les liposomes contiennent des phospholipides possédant une longueur de chaîne acyle contenant au moins 14 atomes de carbone, de préférence au moins 16 atomes de carbone.

4. Composition antiseptique selon la revendication 2, **caractérisée en ce que** les phospholipides naturels trouvent leur origine dans les oeufs ou dans les fèves de soja.

5. Composition antiseptique selon l'une quelconque des revendications précédentes, **caractérisée en ce que** le polyhexanide présente un poids moléculaire de 1500 à 15.000 g/mol et/ou un degré de polymérisation de 12 à 16.

6. Composition antiseptique selon l'une quelconque des revendications précédentes, **caractérisée en ce que** le milieu aqueux est un tampon qui est choisi parmi le groupe qui est constitué par un tampon PBS, un tampon Tris et un tampon HEPES.

7. Composition antiseptique selon l'une quelconque des revendications précédentes, **caractérisée en ce que** la valeur de pH du milieu aqueux s'élève de 6 à 8, de préférence de 7,0 à 7,5.

8. Composition antiseptique selon l'une quelconque des revendications précédentes, **caractérisée en ce que** les liposomes contiennent du cholestérol, de préférence en une quantité s'élevant jusqu'à 50 mol %, de manière particulièrement préférée en une quantité de 15 à 20 mol %, chaque fois rapportés à la totalité des lipides.

9. Composition antiseptique selon l'une quelconque des revendications précédentes, **caractérisée en ce que** les liposomes contiennent de la vitamine E de préférence en une quantité s'élevant jusqu'à 40 mol %, de manière particulièrement préférée en une quantité de 20 mol %, chaque fois rapportés à la totalité des lipides.

10. Composition antiseptique selon l'une quelconque des revendications précédentes, **caractérisée en ce que** les liposomes présentent une dimension moyenne de 50 à 800 nm, de préférence de 150 à 500 nm.

11. Composition antiseptique selon l'une quelconque des revendications précédentes, **caractérisée en ce qu'**elle est présente sous la forme d'une suspension, d'une émulsion, d'une lotion, d'une teinture, d'un spray, d'un gel, d'une crème ou d'un onguent.

12. Procédé pour la préparation d'une composition antiseptique à base d'une substance active antimicrobienne incorporée dans des liposomes, les liposomes ne contenant aucun lipide comprenant des groupes de tête anioniques, et l'intérieur des liposomes présentant un milieu aqueux dans lequel est contenu du polyhexanide à titre de substance active antimicrobienne, **caractérisé en ce que**
- on procède à une injection, dont la pression est contrôlée, d'une phase lipidique éthanolique dans une phase aqueuse contenant la substance active antimicrobienne choisie parmi le groupe des biguanides ;
- on dilue la phase aqueuse avec un tampon une fois que l'on a obtenu des vésicules ; et
- on élimine la substance active qui n'a pas été incorporée.

13. Procédé selon la revendication 12, **caractérisé en ce que** la phase lipidique comprend des phospholipides qui sont choisis parmi le groupe des phospholipides naturels ou synthétiques, de préférence parmi le groupe qui est constitué par la phosphatidylcholine, la phosphatidyl-éthanolamine, la dimyristoylphosphatidylcholine et par leurs mélanges réciproques.

14. Procédé selon la revendication 13, **caractérisé en ce que** les phospholipides sont choisis parmi le groupe des phospholipides qui présentent une longueur de chaîne acyle contenant au moins 14 atomes de carbone, de préférence parmi le groupe des phospholipides qui présentent une longueur de chaîne acyle d'au moins 16 atomes de carbone.

15. Procédé selon la revendication 13, **caractérisé en ce que** les phospholipides naturels trouvent leur origine dans les oeufs ou dans les fèves de soja.

16. Procédé selon l'une quelconque des revendications de 12 à 15, **caractérisé en ce que** le polyhexanide est choisi parmi le groupe des polyhexanides qui présentent un poids moléculaire de 1500 à 15.000 g/mol et/ou un degré de polymérisation de 12 à 16.

17. Procédé selon l'une quelconque des revendications 12 à 16, **caractérisé en ce que** le milieu aqueux est un tampon qui est choisi parmi le groupe qui est constitué par un tampon PBS, un tampon Tris et un tampon HEPES.

18. Procédé selon l'une quelconque des revendications 12 à 17, **caractérisé en ce que** la valeur de pH du milieu aqueux s'élève de 6 à 8, de préférence de 7,0 à 7,5.

19. Procédé selon l'une quelconque des revendications 12 à 18, **caractérisé en ce que** la phase lipidique contient du cholestérol, de préférence en une quantité s'élevant jusqu'à 50 mol %, de manière particulièrement préférée en une quantité de 15 à 20 mol %, chaque fois rapportés à la totalité des lipides.

20. Procédé selon l'une quelconque des revendications 12 à 19, **caractérisé en ce que** la phase lipidique contient de la vitamine E de préférence en une quantité s'élevant jusqu'à 40 mol %, de manière particulièrement préférée en une quantité de 20 mol %, chaque fois rapportés à la totalité des lipides.

21. Utilisation d'une composition antiseptique selon l'une quelconque des revendications 1 à 11, pour la fabrication d'un revêtement pour des blessures ou des plaies.

22. Utilisation selon la revendication 21, **caractérisée en ce qu'**on applique la composition sur la matière de support du revêtement pour des blessures ou des plaies par arrosage, par pulvérisation ou par imprégnation.

23. Utilisation selon la revendication 22, **caractérisé en ce qu'**on mélange une solution polymère avec la composition et on élimine le solvant en tout ou en partie par séchage ou par lyophilisation.

24. Revêtement pour des blessures ou des plaies **caractérisées en ce qu'**il comprend une composition antiseptique selon l'une quelconque des revendications 1 à 11.

25. Revêtement pour des blessures ou des plaies selon la revendication 24, **caractérisé en ce qu'**il est à base de cellulose, de dérivés de la cellulose tels que les carboxyméthylcelluloses, d'alginates, de chitosan, d'amidon, de collagène, de polyacrylates, de polyuréthane ou de mélange des composés susmentionnés, à titre de matière de support.

26. Revêtement pour des blessures ou des plaies selon la revendication 24 ou 25, **caractérisé en ce qu'**il est présent sous la forme d'un hydrogel, d'un hydrocolloïde, d'une éponge, d'une feuille mince, d'une membrane, d'un non tissé, d'un tissu, d'un tricot, d'un ruban de carde ou d'un tamponnement.

27. Revêtement pour des blessures ou des plaies selon l'une quelconque des revendications 24 à 26, **caractérisé en ce que** le revêtement pour des blessures ou des plaies contient le biguanide en une quantité de 0,01 à 1,0 % en poids, rapportés au poids à sec du revêtement.
